# EUROPÄISCHE PATENTANMELDUNG

(11) **EP 0 709 365 A1**
(43) Veröffentlichungstag der Anmeldung: **01.05.1996**
(21) Anmeldenummer: 95115848.4
(22) Anmeldetag: 09.10.1995
(51) Int. Cl.: C07C 215/80, C07F 7/08, A61K 7/13

(54) **Neue substituierte 2,4-Diaminophenole, Verfahren zu ihrer Herstellung und Haarfärbemittel**

(30) Priorität: 27.10.1994 DE 4438129
(71) Anmelder: Hans Schwarzkopf GmbH, D-22763 Hamburg (DE)
(72) Erfinder: Akram, Mustafa, Dr., D-22457 Hamburg (DE); Wolff, Wolfgang, D-22941 Bargteheide (DE); Bittner, Andreas, Dr., D-63071 Offenbach (DE); Kobs, Uwe, Dr., D-46128 Oberhausen (DE)

(57) **Zusammenfassung**

Die Erfindung betrifft Verbindungen der allgemeinen Formel (I) sowie ihre Salze mit anorganischen und organischen Säuren, 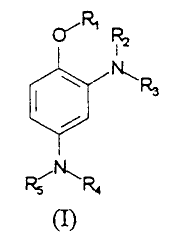
worin R₁ ein Wasserstoffatom oder eine Trimethylsilylgruppe, R₂, R₃, R₄ und R₅ unabhängig voneinander ein Wasserstoffatom, eine (C₁-C₄)-Alkylgruppe, eine Hydroxy-(C₂-C₃)-alkylgruppe, eine Alkoxy-(C₂-C₃)-alkylgruppe, eine Amino-(C₂-C₃)-alkylgruppe, eine 2,3-Dihydroxypropylgruppe ist und wobei R₁, R₂, R₃, R₄ und R₅ nicht gleichzeitig Wasserstoffatome darstellen sowie Verfahren zu ihrer Herstellung und Haarfärbemittel.
Hervorzuheben sind Haarfärbemittel auf der Basis der Verbindungen der allgemeinen Formel (I) in Form einer Creme, Lösung, Schaum oder Gels, die mindestens eine Verbindung der allgemeinen Formel (I) allein oder mit mindestens einem Entwickler enthalten, und einen Oxydationskatalysator für die Bildung einer Oxydationsfärbung durch Luftsauerstoff aufweist, wobei der Oxydationskatalysator aus einem Übergangsmetallsalz oder Übergangsmetallkomplex, insbesondere Kupfer(II)-chlorid, -sulfat, -acetat, allein oder als Addukt mit Ammoniak, Ethylendiamin, Phenanthrolin, Triphenylphosphin, 1,2-Diphenylphosphinoethan, 1,3-Diphenylphosphinopropan oder Aminosäuren einzeln oder im Gemisch besteht, wodurch die zu färbenden Haare besonders geschont werden.

## Beschreibung

Die Erfindung betrifft neue substituierte 2,4-Diaminophenole, Verfahren zu ihrer Herstellung und Haarfärbemittel gemäß den Ansprüchen 1 bis 9.
In großem Umfang werden zum Färben von keratinischen Fasern sogenannte Oxidationsfarben eingesetzt, da diese im Färbeergebnis intensive Farben von hoher Farbechtheit liefern. Unter den anwendungstechnischen Randbedingungen (tiefe Färbetemperatur und kurze Färbedauer) ergeben sie intensive Farben mit guten Echtheiten. Dabei werden die Farbstoffe während des Färbevorgangs durch die oxidative Kupplung einer Entwicklerkomponente und einer Kupplerkomponente gebildet. Derartige Kuppler- und Entwicklerkomponenten zur Erzielung verschiedenster Farbtöne, im folgenden Haarfarbstoffzwischenprodukte bezeichnet, sind in der Literatur (K. Venkataraman, The Chemistry of Synthetic Dyes, Vol. V, Academic Press, *1971*; F. Brody und M. S. Burns, J. Soc. Cosmet. Chem. **19**, 361 - 379, *1968*; H. Husemeyer, J. Soc. Cosmet. Chem. **25**, 131 - 138, *1974*) zahlreich beschrieben.
Obwohl die oxidative Kupplung, d. h. die Entwicklung der Färbung, im Prinzip auch durch Luftsauerstoff erfolgen kann, welche aber in aller Regel zu langsam erfolgt und/oder zu ungleiche Färbeergebnisse liefert, werden in der Regel chemische Oxidationsmittel eingesetzt. Bevorzugt werden dabei Oxidationsmittel auf der Basis von Wasserstoffperoxid, neben Wasserstoffperoxid selbst auch Additionsverbindungen mit Harnstoff Melamin oder Alkaliperborat, eingesetzt.
Für die praktische Anwendung als Haarfärbemittel werden die Haarfarbstoffzwischenprodukte in eine geeignete kosmetische Grundlage eingearbeitet, die je nach Art des gewünschten Endproduktes eine Lösung, eine Creme, einen Schaum oder ein Gel sein kann. Diese Zubereitung wird unmittelbar vor der Applikation am Haar mit dem Oxidationsmittel vermischt.
Wie schon erwähnt, kann das Oxidationsmittel zur Entwicklung der Färbung grundsätzlich auch Luftsauerstoff sein. Die Erfahrung lehrt aber, daß die Dauer der Farbbildung oder die dafür nötigen Temperaturen, um diese Zeit zu verkürzen, für eine kosmetische Anwendung am Menschen zu hoch sind. Den Erfindern ist kein kommerzielles Produkt bekannt geworden welches Oxidationskatalysatoren enthält.
Es wurde nun überraschend gefinden, daß sich ein solches Haarfärbemittel schaffen läßt, indem man eine Zusammensetzung wählt, bei der, ausgehend von einer üblichen Grundlage, die farbgebenden Komponenten neue substituierte 2,4-Diaminophenole der allgemeinen Formel (I) oder bekannte, aber zur Luftoxidation zu langsam reagierende Kuppler sind, die durch den Zusatz eines Katalysator in die Färbemasse brauchbare Färbungen durch Luftoxidation auf dem Haar liefert.
Für die Haarfärbemittel gemäß den Ansprüchen 8 und 9, die andere spezielle Ausführungsformen der Erfindung darstellen, können alle bekannten Kuppler- und Entwickler-Kombinationen Verwendung finden. Bevorzugte Kuppler und Entwickler gemäß den Ansprüchen 8 und 9 sind folgende Kombinationen:
1. p-Toluylendiamin, Resorcin, m-Aminophenol, 4-Chlorresorcin
2. p-Toluylendiamin, 2-Methylresorcin, 4-Chlorresorcin, 2-Amino-3-hydroxypyridin
3. p-Toluylendiamin, Resorcin, m-Aminophenol, p-Aminophenol, 2-Hydroxy-4-aminotoluol
4. 3-Methyl-4-aminophenol, m-Aminophenol, 2-Hydroxy-4-aminotoluol, 2-Amino-3-hydroxypyridin
5. 3-Methyl-4-aminophenol, 2-Methylresorcin, m-Aminophenol, p-Toluylendiamin, 2-Hydroxy-4-aminotoluol, 2-Amino-3-hydroxypyridin
Haarfärbemittel gemäß den Ansprüchen 8 und 9 werden durch die Beispiele 4, 5, 6 und 8 verdeutlicht.

Eine spezielle bevorzugte Ausführungsform der Erfindung ist daher ein Haarfärbemittel auf der Basis von Oxidationsfarben, gekennzeichnet durch
1. das Vorliegen eines einkomponentigen Haarfärbesystems,
2. den Verzicht auf eine Vorbehandlung des Haares mit Katalysatoren,
3. den Zusatz eines Katalysators zur Färbemasse,
4. den Verzicht auf Wasserstoffperoxid als Oxidationsmittel,
5. die Verwendung von bekannten, bisher zur Luftoxidation nicht befähigten Kupplern,
6. die Verwendung von neuen substituierten 2,4-Diaminophenolen,
7. Übergangsmetallsalze und Übergangsmetallkomplexe, insbesondere Kupfer(II)-chlorid, -sulfat, -acetat, allein oder als Addukt mit Ammoniak, 1,2-Ethylendiamin, Phenanthrolin, Triphenylphosphin, 1,2-Diphenylphosphinoethan, 1,3-Diphenylphosphinopropan oder Aminosäuren, z. B. seien ohne einschränken zu wollen genannt: Glycin, Leucin, Methionin, Alanin, Phenylalanin und Prolin als Katalysator zwecks Zusatz zur Färbemasse.
Bei der Grundmasse des erfindungsgemäßen Haarfärbemittels handelt es sich je nach Art des gewünschten Endproduktes beispielsweise um ein Gel, eine Creme, einen Schaum oder eine tensidhaltige Lösung. Die Grundmasse besteht aus dem Stand der Technik bekannten und für die Anwendung auf dem Haar geeigneten Bestandteile in für diese Zwecke üblichen Mengen. Die Bestandteile solcher Grundlagen sind z. B.
1. Netz- und Emulgiermittel
2. Verdickungsmittel
3. Reduktionsmittel
4. Parfümöle
5. haarpflegende Zusätze und
6. Lösungsmittel, z. B. Wasser oder niedere Alkohole
Das Färben von Haaren mit dem erfindungsgemäßen Haarfärbemittel erfolgt, indem die Komponente, mit Wasser verdünnt oder unverdünnt, auf das Haar aufgetragen und verteilt wird. Die erforderliche Menge an Haarfärbemittel ist, wie dem Fachmann bekannt ist, von der Länge des zu färbenden Haares abhängig. Die Anwendungstemperaturen liegen vorzugsweise in einem Bereich von 15 bis 40 °C und die Einwirkungszeit vorzugsweise zwischen 5 und 45 Minuten, insbesondere 10 bis 35 Minuten. Das Haarfärbemittel wird dann durch Ausspülen entfernt, das Haar gegebenenfalls mit einem Shampoo nachgewaschen und nochmals gespült und dann getrocknet.
Das erfindungsgemäße Haarfärbemittel ist in der speziellen bevorzugten Ausführungsform im Vergleich zu bisher bekannten Haarfärbemitteln einfacher in der Anwendung, milder und verträglicher durch die Luftoxidations-Farbentwicklung in Anwesenheit des in der Färbemasse als Zusatz enthaltenen Katalysators, den Verzicht auf Wasserstoffperoxid, ohne daß Einbußen beim Färbeergebnis hingenommen werden müssen.
Die Umsetzung der Verbindung der Formel (IIa) oder (IIb) mit einem Chlorameisensäure-2-chlorethylester oder Chlorameisensäure-3-chlorpropylester erfolgt in Anlehnung an die bekannte selektive Hydroxyalkylierung eines Amins mit Chlorameisensäurechloralkylesters mit anschließender basischer Behandlung der Chloralkylcarbamate (vgl. Otto, J. Prakt. Chem. **44**, 15, *1890*; R. Adams und J. B. Segur, J. Am. Chem. Soc. **45**, 785, *1923*). J. S. Pierce und R. Adams, J. Am. Chem. Soc. **45**, 790, *1923* beschreiben ausführlich die Umsetzung des Chlorameisensäurechloralkylesters mit primären aromatischen Aminen. Zur Bildung der Verbindungen der allgemeinen Formel (IIIa) und (IIIb) wird die Verbindung der Formel (IIa) oder (IIb) in einem inerten organischen Lösungsmittel, z. B. Dioxan, C₁- bis C₄-Alkohole, Dimethylformamid. Tetrahydrofuran, Toluol, Chlorbenzol, Methylethylketon, 1,2-Dimethoxyethan, Methyl-*tert*.-butylether oder Diethylenglykoldimethylether vorgelegt und auf eine Temperatur zwischen Raumtemperatur und Rückflußtemperatur, vorzugsweise zwischen 30 °C und Rückflußtemperatur erhitzt. Anschließend wird einer der genannten Chlorameisensäurechloralkylester in äquimolarer Menge oder in geringem Überschuß zudosiert. Gegebenenfalls können die Lösungsmittel mit Wasser kombiniert werden. Dabei kann ein säurebindendes Mittel entweder mit vorgelegt oder parallel zum bereits genannten Chlorameisensäurechloralkylester zugefügt werden. Als säurebindende Mittel kommen Basen wie Alkalihydroxide, -hydrogencarbonate, -carbonate, Erdalkalioxide, -hydroxide, -hydrogencarbonate und -carbonate sowie tertiäre organische Amine in Betracht.
Die Reaktionsdauer beträgt zwischen 1 und 12 Stunden.
Nach vollständiger Umsetzung werden die Carbamate isoliert, indem a) Wasser oder Eis oder ein Gemisch von Eis und Wasser und der Ansatz kaltgerührt wird oder b) die anorganischen Salze abfiltriert werden und das Lösungsmittel teilweise oder ganz abdestilliert wird, gegebenenfalls unter Kühlung, Z. B. durch Zugabe von Eis, wodurch die gebildeten Carbamate der Formel (IIIa) oder (IIIb) in fester Form nahezu quantitativ ausfallen.
Durch Behandeln mit starken Basen - hier kommen Alkali- oder Erdalkalihydroxide in Betracht, vorzugsweise wird 10 bis 50 %ige Natron- oder Kalilauge eingesetzt - werden die Carbamate der allgemeinen Formel (IIIa) oder (IIIb) in die Hydroxyalkylverbindungen der Formel (IVa) oder (IVb) überführt.
Dabei sind zwei Verfahrensweisen zweckmäßig:
a) Das Carbamat der Formel (IIIa) oder (IIIb) wird in Wasser oder einem organischen Lösungsmittel, z. B. einem C₁- bis C₄-Alkohol, einem wassermischbaren Ether oder Mischungen davon vorgelegt, bei Raumtemperatur wird dann ungefähr die berechnete Menge Lauge, das sind 4 Mol Lauge pro Mol Carbamat, zudosiert und es wird bis zur vollständigen Umsetzung nachgerührt, wobei ggf. bis zum Rückfluß erhitzt oder Lauge nachgesetzt werden kann.
b) Man legt die Lauge, die mit den genannten Lösungsmitteln verdünnt sein kann, vor, dosiert das Carbamat mit der allgemeinen Formel (IIIa) oder (IIIb) in reiner Form oder gelöst in einem der genannten organischen Lösungsmittel bei einer Temperatur zwischen Raumtemperatur und ca. 70 °C zu und rührt dann bis zur vollständigen Umsetzung nach.
Bei beiden Varianten kann die Reaktionslösung, die einen pH-Wert von ca. 12 bis 14 aufweist, zur Aufarbeitung durch Zusatz einer organischen oder anorganischen Säure auf einen pH-Wert von etwa 5 bis 10 abgestumpft werden. Anschließend trennt man die Salze ab, setzt gegebenenfalls Wasser zu und isoliert das Produkt der allgemeinen Formel (IVa) oder (IVb) nach Entfernen des organischen Lösungsmittels.

Bei den beiden vorstehenden Verfahrensweisen wird durch Zusatz von etwa 25 bis 30 Gew.-% eines der vorstehend genannten organischen Lösungsmittels in den wäßrigen Reaktionsansatz die Reaktionsdauer deutlich verkürzt, wobei die anorganischen Salze noch im Reaktionsmedium gelöst bleiben. Für die Umsetzung werden etwa 1 bis 12 Stunden benötigt.
Die Herstellung der Verbindungen der allgemeinen Formel (I) kann durch Reduktion der Verbindungen der allgemeinen Formel (Va) oder (Vb), gegebenenfalls nach Alkylierung oder Oxalkylierung, mit unedlen Metallen oder durch katalytische Reduktion erfolgen.
Bei der katalytischen Reduktion werden übliche Katalysatoren, z. B. Raney-Nickel, Palladium auf Aktivkohle oder Platin auf Aktivkohle eingesetzt. Die Reaktionstemperatur liegt zwischen Raumtemperatur und 120 °C, vorzugsweise zwischen 35 und 100 °C, der Druck liegt zwischen Normaldruck und 100 bar, vorzugsweise zwischen 20 und 70 bar. Als Lösungsmittel finden übliche Lösungsmittel wie Wasser, Toluol, Eisessig, niedere Alkohole oder Ether Verwendung. Nach erfolgter Reduktion und Abtrennung des Katalysators kann das Produkt der allgemeinen Formel (I), gegebenenfalls nach Alkylierung oder Oxalkylierung, durch Abziehen des Lösungsmittels unter einem Schutzgas in freier Form isoliert werden. Als Alkylierungsmittel haben sich die bekannten Verbindungen Dimethyl- und Diethylsulfat und als Oxalkylierungsmittel die bekannten Verbindungen Ethylenoxid und Propylenoxid bewährt. Das Produkt nach der allgemeinen Formel (I) wird vorzugsweise unter einem Schutzgas durch Zugabe einer ungefähr äquivalenten Menge einer Säure in ein Salz überführt, das entweder direkt ausfällt oder nach Abzug des Lösungsmittels erhalten wird.
Als anorganische Säuren sind z. B. Salzsäure, Schwefelsäure, Phosphorsäure und als organische Säuren Essigsäure, Propionsäure, Milchsäure oder Citronensäure zur Salzbildung geeignet.
Die folgenden Beispiele sollen die Erfindung beschreiben und erläutern, ohne sie darauf zu beschränken zu wollen.

### A. Herstellung der Katalysatoren

### Beispiel A. 1

### Darstellung von Kupfer(II)-glycinat

In 100 mL heißem Methanol werden 1.8 g (9.8 mMol) Kupfer(II)-acetat gelöst und eine Lösung von 1.5 g (20 mMol) Glycin in 50 mL Methanol unter Rühren zugegeben. Die Lösung trübt sich durch ausgefallenes Produkt. Man rührt noch zwei Stunden bei fallender Temperatur, filtriert das ausgefallene Produkt ab und trocknet es.
- Ausbeute:: 2.49 g (96 % d. Th.)
- Schmp.:: >210 °C

### Beispiel A.2

### Darstellung von Kupfer(II)-1,2-diaminoethan-chlorid

In 300 mL heißem Methanol werden 26.9 g (160 mMol) Kupfer(II)-chlorid-Dihydrat gelöst und eine Lösung von 9.6 g (160 mMol) 1,2-Diaminoethan in 100 mL Methanol unter Rühren zugegeben. Die Lösung trübt sich durch ausgefallenes Produkt. Man rührt noch eine Stunde bei Rückfluß nach. Die Mischung läßt man unter Rühren abkühlen, filtriert das ausgefallene Produkt ab und trocknet es.
- Ausbeute:: 12.5g (40% d. Th.)
- Schmp.:: >210 °C

### B. Herstellung neuer Kuppler

Alle hergestellten Verbindungen sind durch IR- oder IR- und ¹H-NMR-Spektren charakterisiert worden.

### Beispiel B.1

### Darstellung von 4-(2-Hydroxyethylamino)-2-aminophenol

### Stufe a)

### (Carbamate aus den Amino-nitrophenolen)

### Darstellung von 4-(2-Chlorethoxycarbonylamino)-2-nitrophenol (B1.a)

In einer Mischung aus 225 mL 1,2-Dimethoxyethan und 25 mL Wasser werden 87.3 g (0.5 Mol) 4-Amino-2-nitrophenol gelöst und 25 g (0.26 Mol) CaCO₃ eingetragen und die Lösung auf ca. 70 °C erhitzt. Unter Rühren tropft man 71.5 g (0.5 Mol) Chlorameisensäure-2-chlorethylester binnen ca. 30 Minuten zu und erhitzt anschließend noch eine Stunde nach. In die Mischung gibt man 312 g Wasser und 312 g Eis, stellt mit konzentrierter Salzsäure einen pH-Wert von 1 ± 0.5 ein. Das ausgefallene Produkt wird abgesaugt, zweimal mit ca. 200 mL Wasser gewaschen und bei 40 °C im Vakuum getrocknet.
- Ausbeute:: 160 g (94 % d. Th.)
- Schmelzpunkt:: 116 - 117 °C

Analog zu dieser Vorschrift werden die in Tabelle 1 aufgeführten Verbindungen hergestellt. In der Tabelle sind in Spalte 1 die laufende Nummer, in Spalte 2 das eingesetzte Aminophenol und in Spalte 3 der zugesetzte Chlorameisensäureester angegeben. Die erhaltene Ausbeute und der Schmelzpunkt der Produkte sind in Spalte 4 und 5 angegeben.

**Tabelle 1**

| Carbamate aus Aminophenolen und Chlorameisensäureestern | | | | |
|---|---|---|---|---|
| Beispiel | ...phenol | Chlorameisensäure-... | Ausbeute [%] | Schmp. [°C] |
| B2.a | 4-Amino-2-nitro... | ...3-chlorpropylester | 87 | 112 - 113 |
| B3.a | 2-Amino-4-nitro... | ...2-chlorethylester | 97 | 190 |
| B4.a | 2-Amino-4-nitro... | ...3-chlorpropylester | 91 | 157 - 159 |

### Stufe b)

### Darstellung von 4-(2-Hydroxyethylamino)-2-nitrophenol (B1.b)

Zu 150 mL Wasser werden 359 g 50 %ige Kalilauge gegeben und 160 g (0.5 Mol) 4-(2-Chlorethoxycarbonylamino)-2-aminophenol (Stufe a) eingetragen. Die Mischung wird drei Stunden bei 40 °C gerührt, anschließend wird der pH-Wert mit Essigsäure auf 6 eingestellt und das Produkt ausgerührt. Das feuchte Produkt wird aus 1,2-Dimethoxyethan umkristallisiert.
- Ausbeute:: 182 g (92 % d. Th.)
- Schmelzpunkt:: 109 - 111 °C
Analog zu dieser Vorschrift werden die in Tabelle 2 aufgeführten Verbindungen hergestellt. In der Tabelle sind in Spalte 1 die laufende Nummer, in Spalte 2 der nach der Umlagerung des eingesetzten Carbamates erhaltene Substituent und in Spalte 3 der zweite Substituent des Phenols angegeben. Die erhaltene Ausbeute und der Schmelzpunkt der Produkte sind in Spalte 4 und 5 aufgeführt.

**Tabelle 2**

| Hydroxyalkylamino-nitrophenole aus den korrespondierenden Carbamaten | | | | |
|---|---|---|---|---|
| Beispiel | ...amino)-... | ...phenol | Ausbeute [%] | Schmp. [°C] |
| B2.b | 4-(3-Hydroxypropyl... | ...2-nitro... | 97 | (Öl) |
| B3.b | 2-(2-Hydroxyethyl... | ...4-nitro... | 92 | 109 - 111 |
| B4.b | 2-(3-Hydroxypropyl... | ...4-nitro... | 86 | 157 - 159 |

### N-Alkylierung der Amino-nitrophenole aus Stufe b)

### Stufe c)

### Darstellung von 4-[Bis(2-hydroxyethyl)-amino]-2-nitrophenol durch Ethoxylierung (B5.c)

In einer Mischung aus 1.6 L Wasser und 1.1 L 1,2-Dimethoxyethan werden 397 g (2 Mol) 4-(2-Hydroxyethylamino)-2-nitrophenol eingetragen, verschließt die Apparatur mit einem Blasenzähler und heizt unter Rühren auf 60 °C an. In diese Lösung leitet man Ethylenoxid so ein, daß durch den Blasenzähler kein Ethylenoxid entweicht. Nach 5 Stunden erhöht man die Temperatur auf 85 °C, setzt 9.2 g Aktivkohle und 4.6 g Celite zu und rührt 15 Minuten weiter. Anschließend filtriert man über eine Nutsche, wäscht den Rückstand mit 100 mL 1,2-Dimethoxyethan/Wasser (1:1 v/v), destilliert aus der Lösung 500 mL 1,2-Dimethoxyethan ab, rührt das Produkt aus, saugt es ab und trocknet es.
(Dieses Produkt erhält man auch ausgehend von 4-Amino-2-nitrophenol nach dieser Vorschrift.)
- Ausbeute:: 260 g (54 % d. Th.)
- Schmelzpunkt:: 103 - 104 °C

Ganz analog zu dieser Vorschrift werden die in Tabelle 3 aufgeführten Verbindungen hergestellt.

**Tabelle 3**

| Ethoxylierungsprodukte aus der Zwischenstufe b) oder des 4-Amino-2-nitrophenols und 2-Amino-4-nitrophenols | | | | |
|---|---|---|---|---|
| Beispiel | ...amino-... | ...phenol | Ausbeute [%] | Schmp. [°C] |
| B6.c | 4-(3-Hydroxypropyl-2-hydroxyethyl... | ...2-nitro... | 45.3 | 99 - 101 |
| B7.c | 2-(3-Hydroxypropyl-2-hydroxyethyl)... | ...4-nitro... | 52.1 | 139 - 141 |
| B8.c | 2-Bis-(2-hydroxyethyl... | ...4-nitro... | 26.8 | 141 - 143 |
| B9.c | 4-Bis-(2-hydroxyethyl... | ...2-nitro... | - | - |

### Darstellung von 4-[(Ethyl-2-hydroxyethyl)-amino]-2-nitrophenol (B10.c)

In einer Mischung aus 150 mL Wasser und 15 mL 1,2-Dimethoxyethan legt man 39.6 g (200 mMol) 4-(2-Hydroxyethylamino)-2-nitrophenol vor und tropft bei 60 °C Innentemperatur 33.9 g (220 mMol) Diethylsulfat so zu, daß der pH-Wert der Lösung durch gleichzeitiges Zutropfen von 10 %iger Natronlauge (ca. 62 mL) zwischen 6 und 8 bleibt. Nach vollständigem Umsatz filtriert man die Lösung durch ein Faltenfilter, kühlt unter Rühren auf 5 °C ab und läßt das Produkt kristallisieren.
- Ausbeute:: 29.1 g (64.1 % d. Th.)
- Schmp.:: 91 - 92 °C

Analog zu dieser Vorschrift werden die in Tabelle 4 aufgeführten Verbindungen hergestellt.

**Tabelle 4**

| Alkylierungsprodukte aus der Zwischenstufe b) oder des 4-Amino-2-nitrophenols und 2-Amino-4-nitrophenols mit Dialkylsulfat | | |
|---|---|---|
| Beispiel | ...amino)-... | ...phenol |
| B11.c | 4-(Methyl-2-hydroxyethyl... | ...2-nitro... |
| B12.c | 4-(Methyl-3-hydroxypropyl... | ...2-nitro... |
| B13.c | 4-(Ethyl-3-hydroxypropyl... | ...2-nitro... |
| B14.c | 2-(Methyl-2-hydroxyethyl... | ...4-nitro... |
| B15.c | 2-(Methyl-3-hydroxypropyl... | ...4-nitro... |
| B16.c | 2-(Ethyl-3-hydroxypropyl... | ...4-nitro... |
| B17.c | 2-(Methyl... | ...4-nitro... |
| B18.c | 2-(Ethyl... | ...4-nitro... |
| B19.c | 4-(Methyl... | ...2-nitro... |
| B20.c | 4-(Ethyl... | ...2-nitro... |

### Beispiel B.21

### Darstellung von 2,4-Dinitrophenoxy-trimethylsilan

In 175 mL 1,2-Dimethoxyethan löst man 46 g (250 mMol) 2,4-Dinitrophenol und 25.3 g (250 mMol) Triethylamin, tropft unter Rühren 27.2 g (250 mMol) Trimethylchlorsilan zu und erhitzt die Mischung nach beendeter Zugabe zwei Stunden auf Rückfluß. Dann filtriert man das ausgefallene Triethylamoniumchlorid ab und tropft die Lösung in 700 mL Wasser. Das ausgefallene Produkt wird abgesaugt, zweimal mit ca. 200 mL Wasser gewaschen und getrocknet.
(Diese Umsetzung ist auch mit Bis(trimethylsilyl)-amin anstelle von Trimethylchlorsilan möglich.)
- Ausbeute:: 39.4g (61.5 % d. Th.)
- Schmp.:: 106 - 107 °C

### Stufe d)

### (Hydrierung der Amino-nitrophenole aus Stufe b oder c und Beispiel B.21)

### Darstellung von 4-(2-Hydroxyethylamino)-2-aminophenol (B.1d)

In einen 0.7 L Autoklaven werden 450 mL Methanol vorgelegt, 96 g (0.5 Mol) 4-(2-Hydroxyethylamino)-2-nitrophenol (Stufe b) gelöst und 5 g Palladium auf Aktivkohle 10 % (Degussa) zugegeben. Nach Verschließen und Inertisieren mit Stickstoff wird bei einem Druck von 20 bar und einer Temperatur von 40 - 45 °C hydriert, bis ca. 11.5 L Wasserstoff (1 bar) aufgenommen wurden. Die warme Lösung wird unter Stickstoff über eine G4-Fritte filtriert, mit 1.1 Mol 70 %iger Schwefelsäure tropfenweise versetzt und unter Rühren abgekühlt. Das ausgefallene Produkt wird abgesaugt, mit 70 mL Methanol gewaschen und getrocknet.
- Ausbeute:: 81.5g (60 % d. Th.)
- Schmp.:: >250 °C

Analog zu dieser Vorschrift werden die in Tabelle 5 aufgeführten Verbindungen hergestellt.
Die Lesart der Tabelle ist die gleiche, wie bei den vorigen.

**Tabelle 5**

| Neue Kuppler durch Hydrierung der Zwischenprodukte aus Stufe b) oder c) | | |
|---|---|---|
| Beispiel | ...amino-... | ...phenol-Sulfat |
| B2.d | 4-(3-Hydroxypropyl)... | ...2-amino... |
| B3.d | 2-(2-Hydroxethyl)... | ...4-amino... |
| B4.d | 2-(3-Hydroxypropyl)... | ...4-amino... |
| B6.d | 4-(3-Hydroxypropyl)-4-(2-hydroxyethyl)... | ...2-amino... |
| B7.d | 2-(3-Hydroxypropyl)-2-(2-hydroxyethyl)... | ...4-amino... |
| B8.d | 2-(Bis-2-hydroxyethyl)... | ...4-amino... |
| B9.d | 4-(Bis-2-hydroxyethyl)... | ...2-amino... |
| B10.d | 4-(Ethyl-2-hydroxyethyl)... | ...2-amino... |
| B11.d | 4-(Methyl-2-hydroxyethyl)... | ...2-amino... |
| B12.d | 4-(Methyl-3-hydroxypropyl)... | ...2-amino... |
| B13.d | 4-(Ethyl-3-hydroxypropyl)... | ...2-amino... |
| B14.d | 2-(Methyl-2-hydroxyethyl)... | ...4-amino... |
| B15.d | 2-(Methyl-3-hydroxypropyl)... | ...4-amino... |
| B16.d | 2-(Ethyl-3-hydroxypropyl)... | ...4-amino... |
| B21.d | O-Trimethylsilyl-2-... | ...4-amino... |

### C. Färbebeispiele

Die erfindungsgemäßen Haarfärbemittel sind wäßrige Mittel. Darunter werden sämtliche Mittel verstanden, die in irgendeiner Weise Wasser enthalten, wie z. B. Cremes, Emulsionen, Gele oder auch einfache Lösungen. Die Zusammensetzungen der Haarfärbemittel stellt eine Mischung der Farbstoffkomponenten mit den für solche kosmetischen Zubereitungen üblichen Zusätzen dar. Übliche Zusätze in Lösungen, Cremes, Emulsionen oder Gelen sind z. B. Lösungsmittel wie Wasser, niedere aliphatische Alkohole, beispielsweise Ethanol, Propanol und Isopropanol, oder Glykole wie Glycerin und Glykolether wie Propylenglykol, weiterhin Netztmittel oder Emulgatoren aus den Klassen der anionischen, kationischen, amphoteren oder nichtionogenen oberflächenaktiven Substanzen wie Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäurester, ferner Verdicker wie höhere Fettalkohole, Stärke, Cellulosederivate, Vaseline, Paraffinöl und Fettsäuren. Die erwähnten Bestandteile werden in den für solche Zwecke üblichen Mengen verwendet, zum Beispiel die Netzmittel und Emulgatoren in Konzentrationen von etwa 0,5 bis 30 Gew. %, während die Verdicker in einer Menge von etwa 0,1 bis 25 Gew. % in den Zubereitungen enthalten sein können.

Je nach Zusammensetzung können die erfindungsgemäßen Haarfärbemittel schwach sauer, neutral oder alkalisch reagieren. Insbesondere weisen sie ein pH-Wert im alkalischen Bereich zwischen 7,5 und 11,5 auf, wobei die Einstellung vorzugsweise mit Ammoniak ertoll. Es können aber auch organische Amine, z. B. Monoethanolamin und Triethanolamin, oder auch anorganische Basen wie Natriumhydroxid und Kaliumhydroxid, Verwendung finden.

Bei Verfahren zur oxidativen Färbung von Haaren werden die Haarfärbemittel dieser Erfindung, welche eine Kombination von in der Haarfärbung bekannten Entwicklersubstanzen mit mindestens einer Verbindung der allgemeinen Formel I als Kupplersubstanz sowie gegebenenfalls zusätzlich bekannte Kupplersubstanzen und direktaufziehende Farbstoffe und gegebenenfalls die bereits erwähnten Katalysatoren enthalten, oder die Haarfärbemittel gemäß den Ansprüchen 8 oder 9, denen vor oder während der Anwendung keine Oxydationsmittel zugefügt werden, auf das Haar aufgetragen. Wahlweise können die Färbemittel gemäß den Ansprüchen 3 bis 6 vor der Applikation auch mit Oxidationsmittel vermischt werden. Als Oxidationsmittel zur Entwicklung der Haarfärbung kommen hauptsächlich Wasserstoffperoxid, beispielsweise als 6 %ige wäßrige Lösung und dessen Additionsverbindungen an Harnstoff Melamin oder Natriumborat sowie Gemische aus derartigen Wasserstoffperoxidanlagerungsverbindungen mit Kaliumperoxodisulfat in Betracht.
Die Anwendungstemperaturen bewegen sich dabei im Bereich von 15 bis 40 C. Nach einer Einwirkungsdauer von ca. 10 Minuten wird das Haarfärbemittel vom zu färbenden Haar durch Spülen entfernt. Hiernach kann das Haar mit einem milden Shampoo nachgewaschen und getrocknet werden.
Die nachfolgenden Beispiele sollen den Erfindungsgegenstand näher erläutern, ohne ihn jedoch, hierauf zu beschränken.

### Beispiel 1

### Haarfärbemittel in Cremeform

- 2,20 g: p-Toluylendiamin-Sulfat
- 2,66 g: 4-(2-Hydroxyethylamino)-2-aminophenol (als Sulfat)
- 1,20g: Ölsäure
- 0,50g: Natriumdithionit
- 6,20 g: Laurylalkoholdiglykolethersulfat, Natriumsalz (28 %ige Lsg.)
- 18,0 g: Cetyl-Stearylalkohol
- 7,50 g: Ammoniak, 25%
Wasser auf 100 g
40 g des vorstehend genannten Haarfärbemittels werden auf 100 %ig ergrautem Haar mittels eines Pinsels aufgetragen. Nach einer Einwirkzeit von 10 Minuten bei Raumtemperatur wird das Färbermittel abgespült und das Haar getrocknet. Das Haar hat einen gleichmäßigen hell- bis mittelblond-cendré Farbcharakter erhalten.

### Beispiel 2

### Haarfärbemittel in Gelform

- 2,20 g: p-Toluylendiamin-Sulfat
- 2,66 g: 4-(2-Hydroxyethylamino)-2-aminophenol (als Sulfat)
- 12,0g: Ölsäure
- 12,0 g: Isopropanol
- 5,00 g: Nonoxynol-4
- 10,0g: Ammoniak, 25 %
- 0,5 g: Natriumsulfit, wasserfrei
Wasser auf 100 g
60 g des vorstehend genannten Färbemittels werden kurz vor dem Gebrauch mit 60 g Wasserstoffperoxid-Lösung, 6 % gemischt. Man läßt das Gemisch 10 Min bei 35 °C auf 100 %ig ergrautes Haar einwirken. Danach wird die Farbmasse ausgespült, nachshampooniert und das Haar getrocknet. Das Haar ist in einem mittelblonden Farbton eingefärbt.

### Beispiel 3

### Haarfärbemittel in Cremeform

- 2,20 g: p-Toluylendiamin-Sulfat
- 2,66 g: 4-(2-Hydroxyethylamino)-2-aminophenol (als Sulfat)
- 1,20g: Ölsäure
- 0,50 g: Natriumdithionit
- 6,20 g: Laurylalkoholdiglykolethersulfat, Natriumsalz (28 %ige Lsg.)
- 18,0 g: Cetyl-Stearylalkohol
- O,20 g: Kupfertetramminsulfat
- 7,50 g: Ammoniak, 25%
Wasser auf 100 g
40 g des vorstehend genannten Haarfärbemittels werden auf 100 %ig ergrautem Haar mittels eines Pinsels aufgetragen. Nach einer Einwirkzeit von 10 Minuten bei Raumtemperatur wird das Färbemittel abgespült und das Haar getrocknet. Das Haar hat einen gleichmäßigen mittelblond-matten Farbcharakter erhalten.

### Beispiel 4

### Haarfärbemittel in Cremeform

- 2,20 g: p-Toluylendiamin-Sulfat
- 1,10g: Resorcin
- 1,20g: Ölsäure
- 0,50 g: Natriumdithionit
- 6,20 g: Laurylalkoholdiglykolethersulfat, Natriumsalz (28 %ige Lsg.)
- 18,0 g: Cetyl-Stearylalkohol
- O,20 g: Kupfertetramminsulfat
- 7,50g: Ammoniak, 25%
Wasser auf 100 g
40 g des vorstehend genannten Haarfärbemittels werden auf 40 %ig ergrautes mittelblondes Haar aufgetragen. Nach einer Einwirkzeit von 15 Minuten bei Raumtemperatur wird das Färbemittel abgespült und das Haar getrocknet. Das Haar hat einen gleichmäßigen goldbraunen Farbton erhalten.

### Beispiel 5

### Haarfärbemittel in Cremeform

- 2,20 g: p-Toluylendiamin
- 1,09 g: m-Aminophenol
- 0,01 g: HC Red No.3
- 2,50 g: Laurylethersulfat Natriumsalz (70 %ige Paste)
- 1,00 g: Ölsäure
- 0,60 g: Natriumsulfit, wasserfrei
- 12,0 g: Cetylalkohol
- 6,00 g: Myristylalkohol
- 1,00 g: Propylenglykol
- 0,20 g: Kupfer(II)-1,2-ethylendiamin-dichlorid
- 10,0g: Ammoniak, 25 %
Wasser auf 100 g
40 g des vorstehend genannten Haarfärbemittels werden auf 70 %ig ergrautes mittelblondes Haar aufgetragen. Nach einer Einwirkzeit von 12 Minuten bei Raumtemperatur wird das Färbemittel abgespült und das Haar getrocknet. Das Haar hat einen gleichmäßigen hellblonden Farbton mit einem aschigen Reflex erhalten.

### Beispiel 6

### Haarfärbemittel in Cremeform

- 2,20 g: p-Toluylendiamin
- 1,23 g: p-Amino-o-cresol
- 0,01 g: HC Red No.3
- 2,50 g: Laurylethersulfat Natriumsalz (70 %ige Paste)
- 1,00g: Ölsäure
- 0,60 g: Natriumsulfit, wasserfrei
- 12,0 g: Cetylalkohol
- 6,00 g: Myristylalkohol
- 1,00 g: Propylenglykol
- 0,20 g: Kupfer(II)-1,10-Phenanthrolin-dichlorid
- 10,0g: Ammoniak, 25 %
Wasser auf 100 g
40 g des vorstehend genannten Haarfäbemittels werden auf mittelblondes Naturhaar aufgetragen. Nach einer Einwirkzeit von 12 Minuten bei Raumtemperatur wird das Färbemittel abgespült und das Haar getrocknet. Das Haar hat einen gleichmäßigen dunkelblond violetten Farbton erhalten.

### Beispiel 7

### Haarfärbemittel in Cremeform

- 2,66 g: 4-(2-Hydroxyethylamino)-2-aminophenol (als Sulfat)
- 1,20g: Ölsäure
- 0,50 g: Natriumdithionit
- 6,20 g: Laurylalkoholdiglykolethersulfat, Natriumsalz (28 %ige Lsg.)
- 18,0 g: Cetyl-Stearylalkohol
- 7,50 g: Ammoniak, 25%
Wasser auf 100 g
40 g des vorstehend genannten Haarfärbemittels werden auf 100% ergrautes Naturhaar aufgetragen. Nach einer Einwirkzeit von 10, 20 und 30 Minuten bei Raumtemperatur wird das Färbemittel abgespült und das Haar getrocknet. Das Haar hat einen gleichmäßigen rötlich violetten Farbton erhalten. Die Farbtiefe hat sich entsprechend der Verweilzeit des Haarfärbemittels aufgebaut.

### Beispiel 8

### Haarfärbemittel in Cremeform

- 2,20 g: p-Phenylendiamin-Sulfat
- 6.20 g: 4-(2-Hydroxyethylamino)-2-aminoanisol-Sulfat
- 1,20g: Ölsäure
- 0,50 g: Natriumdithionit
- 6,20 g: Laurylalkoholdiglykolethersulfat, Natriumsalz (28 %ige Lsg.)
- 18,0 g: Cetyl-Stearylalkohol
- 0,20 g: Kupfer(II)-glycinat
- 7,50 g: Ammoniak, 25%
Wasser auf 100 g
40 g des vorstehend genannten Haarfärbemittels werden auf 100% ergrautes Naturhaar aufgetragen. Nach einer Einwirkzeit von 10 Minuten bei Raumtemperatur wird das Färbemittel abgespült und das Haar getrocknet. Das Haar hat einen gleichmäßigen dunkel-blaugrauen Farbton erhalten.

### Beispiel 9

### Haarfärbemittel in Cremeform

- 2,66 g: 4-(2-Hydroxyethylamino)-2-aminophenol (als Sulfat)
- 1,20g: Ölsäure
- 0,50 g: Natriumdithionit
- 6,20 g: Laurylalkoholdiglykolethersulfat, Natriumsalz (28 %ige Lsg.)
- 18,0 g: Cetyl-Stearylalkohol
- 0,20 g: Kupfer(II)-glycinat
- 7,50 g: Ammoniak, 25%
Wasser auf 100 g
40 g des vorstehend genannten Haarfärbemittels werden auf 100% ergrautes Naturhaar aufgetragen. Nach einer Einwirkzeit von 10, 20 und 30 Minuten bei Raumtemperatur wird das Färbemittel abgespült und das Haar getrocknet. Das Haar hat einen gleichmäßigen mittelblonden Farbton mit violettem Reflex erhalten. Die Farbtiefe hat sich entsprechend der Verweilzeit des Haarfärbemittels aufgebaut.

## Patentansprüche

1. Verbindungen der allgemeinen Formel (I) sowie ihre Salze mit anorganischen und organischen Säuren, 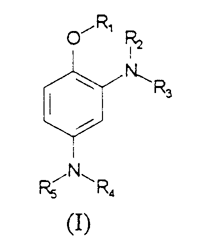 worin R₁ ein Wasserstoffatom oder eine Trimethylsilylgruppe, R₂, R₃, R₄ und R₅ unabhängig voneinander ein Wasserstoffatom, eine (C₁-C₄)-Alkylgruppe, eine Hydroxy-(C₂-C₃)-alkylgruppe, eine Alkoxy-(C₂-C₃)-alkylgruppe, eine Amino-(C₂-C₃)-alkylgruppe, eine 2,3-Dihydroxypropylgruppe ist und wobei R₁, R₂, R₃, R₄ und R₅ nicht gleichzeitig Wasserstoffatome darstellen.

2. Verfahren zur Herstellung der Verbindungen der allgemeinen Formel (I), sowie ihre Salze mit anorganischen und organischen Säuren, 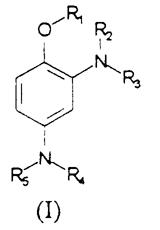 worin R₁ ein Wasserstoffatom oder eine Trimethylsilylgruppe, R₂, R₃, R₄ und R₅ unabhängig voneinander ein Wasserstoffatom, eine (C₁-C₄)-Alkylgruppe, eine Hydroxy-(C₂-C₃)-alkylgruppe, eine Alkoxy-(C₂-C₃)-alkylgruppe, eine Amino-(C₂-C₃)-alkylgruppe, eine 2,3-Dihydroxypropylgruppe und wobei R₁, R₂, R₃, R₄ und R₅ nicht gleichzeitig Wasserstoffatome darstellen, dadurch gekennzeichnet, daß man eine Verbindung der allgemeinen Formel (IIa) oder (IIb), wobei R1 die unter Anspruch 1 genannten Bedeutungen hat, 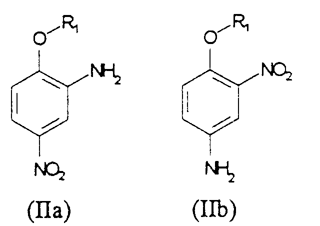 in einem inerten Lösungsmittel mit Chlorameisensäure-2-chlorethylester oder Chlorameisensäure-3-chlorpropylester zu den Verbindungen der allgemeinen Formel (IIIa) oder (IIIb), wobei n = 2 oder n = 3 ist, umsetzt, 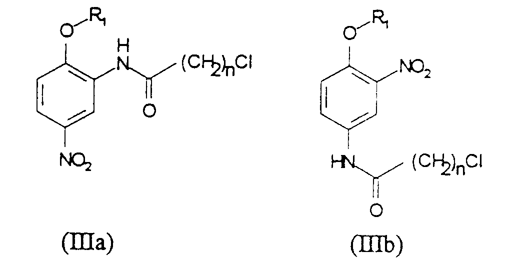 die in einem Lösungsmittel mit Basen zu den Verbindungen der allgemeinen Formel (IVa) oder (IVb), wobei n = 2 oder n = 3 ist, umgesetzt werden, die mit bekannten 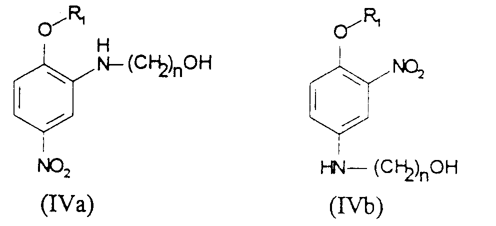 Alkylierungsmitteln oder Oxalkylierungsmitteln zu den Verbindungen der allgemeinen Formel (Va) oder (Vb), wobei n = 2 oder n = 3 ist und R₂ bis R₅ die die bereits angegebenen Bedeutungen haben, umgesetzt werden, die nach Reduktion und gegebenenfalls nach weiterer Alkylierung oder Oxalkylierung die Verbindungen der allgemeinen Formel (I) ergeben 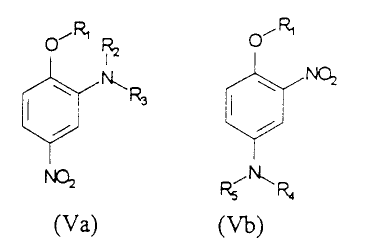 und diese gegebenenfalls mit einer anorganischen oder organischen Säure in ihr Salz überführt.

3. Haarfärbemittel auf der Basis der Verbindungen der allgemeinen Formel (I) in Form einer Creme, Lösung, Schaum oder Gels die mindestens eine Verbindung der allgemeinen Formel (I) allein oder mit mindestens einem Entwickler enthalten.

4. Haarfärbemittel auf der Basis der Verbindungen der allgemeinen Formel (I) in Form einer Creme, Lösung, Schaum oder Gels die mindestens eine Verbindung der allgemeinen Formel (I) allein oder mit mindestens einem Entwickler enthalten, bei dem die Oxidationsfärbung durch Zugabe eines Oxidationsmittels bewirkt wird.

5. Haarfärbemittel auf der Basis der Verbindungen der allgemeinen Formel (I) in Form einer Creme, Lösung, Schaum oder Gels die mindestens eine Verbindung der allgemeinen Formel (I) allein oder mit mindestens einem Entwickler enthalten, bei dem die Oxidationsfärbung durch Zugabe eines Oxidationsmittels und eines Oxydationskatalysators bewirkt wird.

6. Haarfärbemittel auf der Basis der Verbindungen der allgemeinen Formel (I) in Form einer Creme, Lösung, Schaum oder Gels die mindestens eine Verbindung der allgemeinen Formel (I) enthalten in Abwesenheit eines Entwicklers.

7. Haarfärbemittel auf der Basis der Verbindungen der allgemeinen Formel (I) in Form einer Creme, Lösung, Schaum oder Gels die mindestens eine Verbindung der allgemeinen Formel (I) allein oder mit mindestens einem Entwickler enthalten, und einen Oxydationskatalysator für die Bildung einer Oxydationsfärbung durch Luftsauerstoff aufweist, wobei der Oxydationskatalysator aus einem Übergangsmetallsalz oder Übergangsmetallkomplex, insbesondere Kupfer(II)-chlorid, -sulfat, -acetat, allein oder als Addukt mit Ammoniak, Ethylendiamin, Phenanthrolin, Triphenylphosphin, 1,2-Diphenylphosphinoethan, 1,3-Diphenylphosphinopropan oder Aminosäuren einzeln oder im Gemisch besteht.

8. Haarfärbemittel, welche durch Luftoxidation eine Haaroxidationsfärbung erzeugt, in Form einer Creme, Lösung, Schaum oder Gels die mindestens einen Kuppler und mindestens einen Entwickler enthalten und als Oxidationskatalysator für Luftoxidation Kupfer(II)-1,2-diaminoethan-chlorid, Kupfer(II)-1,10-phenanthrolin-chlorid, Kupfer(II)-tetramminsulfat, Kupfer(II)-glycinat einzeln oder im Gemisch enthalten.

9. Haarfärbemittel nach Anspruch 8, in dem der Gehalt an Katalysator von 0.05 bis 0.5 Gewichtsprozent bezogen auf das Gewicht des Haarfärbemittels beträgt.
